# EUROPEAN PATENT SPECIFICATION

(11) **EP 1 546 069 B1**
(45) Date of publication and mention of the grant of the patent: **27.05.2009**
(21) Application number: 03753462.5
(22) Date of filing: 23.09.2003
(51) Int. Cl.: C07C 2/32, B01J 8/18

(54) **PROCESS FOR MAKING A LINEAR ALPHA-OLEFIN OLIGOMER USING A HEAT EXCHANGER**
VERFAHREN ZUR HERSTELLUNG EINES LINEAREN OLEFIN-OLIGOMER UNTER VERWENDUNG EINES WÄRMETAUSCHERS
PROCEDE DE FABRICATION D'UN OLIGOMERE D'ALPHA-OLEFINE LINEAIRE AU MOYEN D'UN ECHANGEUR THERMIQUE

(30) Priority: 25.09.2002 US 413278 P
(43) Date of publication of application: 29.06.2005
(73) Proprietor: SHELL INTERNATIONALE RESEARCH MAATSCHAPPIJ B.V., 2596 HR Den Haag (NL)
(72) Inventor: ARNOLDY, Peter, NL-1031 CM Amsterdam (NL); DE BOER, Eric, Johannes, Maria, NL-1031 CM Amsterdam (NL); MOENE, Robert, NL-1031 CM Amsterdam (NL); VAN ZON, Arie, NL-1031 CM Amsterdam (NL); UNGER, Phillip, Edward, Houston, TX 77080 (US)
(86) International application number: PCT/EP2003/010709
(87) International publication number: WO 2004/029012

(56) References cited:
- WO-A-00/15646
- WO-A-02/00339
- WO-A-02/06192
- US-A- 3 968 090
- US-A- 5 541 270

## Description

The invention pertains to a process for making a linear alpha-olefin oligomer in a reactor comprising a liquid and a gas phase, comprising the steps of catalytically oligomerizing ethylene in the presence of an iron complex of a 2,6-bis(arylimino)pyridine derivative, to the alpha-olefin oligomer with an average molecular weight between 50 and 350 under release of heat, and removing the heat with a heat exchanger.

Various processes are known for the production of higher linear alpha olefins ((for example D. Vogt, Oligomerisation of ethylene to higher a-olefins in Applied Homogeneous Catalysis with Organometallic Compounds, Ed. B. Cornils, W.A. Herrmann, 2nd Edition, Vol. 1, Ch. 2.3.1.3, page 240-253, Wiley-VCH 2002). These commercial processes afford either a Poisson or Schulz-Flory oligomer product distribution. In such a process, a wide range of oligomers is typically made. In WO 02/00339, WO 02/12151, WO 02/06192, WO 02/28805, WO 01/58874, and WO 99/02472 novel Fe-based ethylene oligomerization catalysts are described that show high activity and high selectivity towards linear alpha-olefins. These catalysts are based on iron complexes of a selected 2,6-pyridinedicarboxaldehyde bisimine or a selected 2,6-diacylpyridine bisimine.

In the present invention the term "bis-(arylimino)-pyridine" is used to describe both classes of ligands. Alpha-olefin oligomers are compounds or mixture of compounds with the general formula H₂C=CH-(CH₂CH₂)ₙH wherein n is an integer of 1 or greater. In such oligomers the alpha-olefin oligomer is usually a mixture of alpha-olefin oligomers with a mean number n from 1 to 20, preferably from 2 to 10. Alpha-olefin oligomers prepared according to the process of the present invention preferably have an average molecular weight between 50 and 350, more preferably between 60 and 280, even more preferably between 80 and 210.

The reaction of ethylene in the presence of the above iron complex is usually run in a well-mixed reactor in the liquid phase, typically using an aprotic organic solvent. This reaction generates a large amount of heat, which should be removed. As described in WO 02/06192 it is preferred to install a plurality of small reactors in combination with several heat exchangers to help provide sufficient cooling capacity for the reactor system. The process temperature, which usually is between about 35°C and about 90°C, more preferably between about 35°C and about 75°C, affects the cost of manufacture of the alpha-olefins in several ways. The higher the temperature the smaller the heat exchangers which have to be applied to the reactor(s), which generally lowers cost. The decay of the active oligomerization catalyst increases with increasing temperature. It is found that maximum volumetric production of alpha-olefins coupled with good absolute productivity of the catalyst usually occurs in the range of about 45°C to about 75°C, so this temperature range is preferred. Finally, the temperature also affects the bubble point pressure, the amount of ethylene in the liquid phase, and the catalyst selectivity. The higher the temperature the higher the pressure needed to maintain catalyst selectivity, which increases capital cost of the manufacturing plant because of, for example, the need for thicker vessels, and larger compressors to attain the higher ethylene pressure. Higher pressure also increases energy costs.

The amount of ethylene (ethene) oligomerization catalyst used in the reaction will preferably be the maximum permitted by the cooling capacity of the reactor(s) and the ethylene mass transfer from the gas to the liquid phase. Catalyst may be added to the first reactor only or to one or more subsequent reactors in series. Differing amounts of catalyst may be added to each reactor. The oligomerization is quite exothermic, about 100 kJ/mole of ethylene oligomerized, and as such cooling will usually be applied to the reactor(s) to maintain the desired process temperature while maintaining high volumetric productivity of the reactor(s).

In the prior art cooling is accomplished by running cooling tubes through the liquid in the interior of one or more of the reactors to cool the contents. Another method of cooling is to have one or more heat exchangers external to the reactors and connected to the reactors by a liquid loop to cool the reactor contents. These external heat exchangers may be typical shell and tube exchangers. The reactors may also be jacketed with a cooling jacket. Some or all of the feeds to some or all of the reactors may be cooled to allow the sensible heat of the ingredients to cool the reactors. All these liquid cooling methods, however, suffer from the disadvantage of wax and polyethylene fouling of the coolers, which necessitates regular shut down of the reactor to allow cleaning of the coolers. Furthermore, wax and polyethylene fouling may increase the paraffinicity of the solvent.

It is therefore an objective of the present invention to devise a process without the above disadvantages. It has now been found that linear alpha-olefin oligomers can be made in a reactor comprising a liquid and a gas phase, comprising the steps of catalytically oligomerizing ethylene in the presence of an iron complex of a 2,6-bis(arylimino)pyridine derivative, to the alpha-olefin oligomer with an average molecular weight between 50 and 350 under release of heat, and removing the heat with a heat exchanger, which is not in direct contact with the liquid phase, using at least part of the gas phase as a coolant medium.

This method provides a cooling system having its cooling elements outside the liquid reaction medium. Since wax and polyethylene have high boiling points, deposit of wax and polyethylene can no longer occur, and fouling of the heat exchanger is effectively prevented.

The heat exchanger according to this invention is of a conventional type, such as a shell- and tube-type, and the like. The heat exchanger is internally cooled with conventional cooling fluids, like water, ammonia, Freon®, and the like. The reaction heat causes the solvents, reactants, and/or reaction products, which are present in the reaction medium, to evaporate and subsequently to be cooled by the heat exchanger, after which it works as a coolant medium for the reactor. The heat exchanger can be placed inside or outside the reactor. When the heat exchanger is placed inside the reactor it is preferred that some condensation occurs on the heat exchanger surface. When the heat exchanger is placed outside the reactor, it is preferred to apply a forced circulation of the reactor coolant medium from the gas phase of the reactor through heat exchanger(s) compressor(s)/pump(s) and optionally a gas-liquid separator back to the liquid phase of the reactor. This will additionally improve the mixing in the reactor. After cooling this reactor coolant medium in this loop, some condensation can occur. This allows application of a separate gas and liquid return to the reactor using a gas-liquid separator. Furthermore, it is possible to deliberately remove (part of) this liquid phase from this gas-liquid separator and route this directly to the product work-up section. Finally, if full condensation occurs, return of this liquid to the reactor can be achieved by a pump instead of a compressor, which lowers costs. This reactor coolant medium is selected from an alkane, alkene, and aromatic compound, and mixtures thereof, preferably propane, n-pentane, isopentane, ethylene, 1-butene, o-, m-, and p-xylene, and toluene, and mixtures thereof.

An additional advantage of the present process is the possibility to apply only one reactor, because the efficiency and the lack of fouling no longer necessitates the use of a plurality of small reactors. This adds considerably to the lowering of costs of the oligomerization process.

The iron complexes of the 2,6-bis(arylimino)pyridine type that can be used in the above process are known in the art, and are described in WO 02/00339, WO 02/12151, WO 02/06192, WO 02/28805, WO 01/58874, and WO 99/02472. Any of these complexes can be used. Best results, however, are obtained with such iron complexes wherein one of the aryl moieties of the 2,6-bis(arylimino)pyridine derivative is 2,6-disubstituted with the group CH₂R or C₂H₅R, wherein R is selected from H, F, and substituted or unsubstituted aryl, preferably selected from H and F, and the other aryl moiety is 2,6-unsubstituted, or wherein both aryl moieties of the 2,6-bis(arylimino)pyridine derivative are 2,6-disubstituted with F or Cl.

Particularly useful are the 2,6-bis(arylimino)pyridine derivatives with the formula: 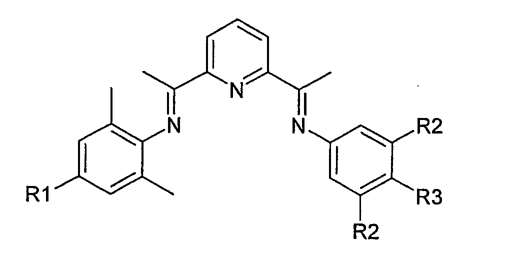 wherein
R1 is H or CH₃;
R2 is H, tert-butyl or phenyl; and
R3 is H, tert-butyl or OR' wherein R' stands for CH₃, Si(CH₃)₃ or eicosyl (C₂₀H₄₁); and

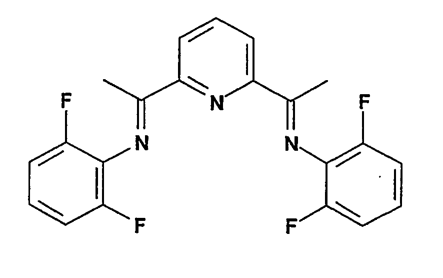

The term "aryl" means an aromatic group, such as phenyl, naphthyl, thienyl, pyridyl, pyrrolyl, and the like. Phenyl is the preferred aryl group. Preferred phenyl groups are substituted with CH₃, tert-butyl, F, or OR' wherein R' stands for CH₃ or Si(CH₃)₃.

In a preferred embodiment an aluminum-based co-catalyst, preferably a methylaluminoxane, is added to the liquid phase. Where a co-catalyst such as an alkylaluminum compound is required or preferred for the active catalyst species, an iron complex of a 2,6-bis(arylimino)pyridine derivative, such as a complex of the 2,6-bis(arylimino)pyridine derivative with FeCl₂, may be reacted with an alkylaluminum compound, preferably an aluminoxane, to form an active ethylene oligomerization species. Specific alkylaluminum compounds include methylaluminoxane (which is an oligomer with the general formula (MeAlO)ₙ), (C₂H₅)₂AlCl, C₂H₅AlCl₂, (C₂H₅)₃Al and ((CH3)₂CHCH₂)₃Al. A particularly preferred aluminoxane is methyl aluminoxane. The ratio of aluminum (as alkylaluminum compound) to iron (as a complex) in the oligomerization may be about 10 to about 10,000.

Another preferred component of the catalyst systems herein is a second co-catalyst compound selected from formula ZnR'₂ wherein each R', which may be the same or different, is selected from hydrogen, optionally substituted C₁-C₂₀ hydrocarbyl, phenyl, Cl, Br, I, SR", NR''₂, OH, OR", CN, NC wherein R", which within the same molecule may be the same or different, is C₁-C₂₀ hydrocarbyl.

In preferred catalyst systems herein, the second co-catalyst compound is ZnR'₂ wherein R' is C₁-C₂₀ hydrocarbyl, more preferably C₁-C₂₀ alkyl, even more preferably C₁-C₆ alkyl. Suitable alkyl groups include methyl, ethyl, propyl, butyl, and the like. It is especially preferred that the R' group is a C₁-C₃ alkyl, especially ethyl.

The second co-catalyst is particularly valuable in combination with the aluminium-based co-catalyst for increasing the selectivity of linear alpha olefins in ethylene oligomerization reactions, and decreasing the amount of unwanted by-products such as branched olefins, internal olefins, 2,2-disubstituted olefins, and dienes.

It has been noted that particularly high selectivity of linear alpha olefins is achieved when the molar ratio of the metal of the aluminium-based co-catalyst to the metal of the second co-catalyst is in the range of from 5:1 to 1:5, preferably from 3:1 to 1:3, more preferably from 2:1 to 1:2 and especially 1:1.

It is possible to add further optional components to the catalyst systems herein, for example, Lewis acids and bases such as those disclosed in WO02/28805.

The active catalyst system may be formed by mixing together the iron complex of a 2,6-bis(arylimino)pyridine derivative or a mixture of the iron acetylacetonate complex and the appropriate 2,6-bis(arylimino)pyridine derivative (ligand), first co-catalyst compound, second co-catalyst compound and any optional additional compounds, preferably in a solvent.

An important item in the capital cost of this manufacturing plant and in its cost of operation is the amount of reactor coolant medium that must be recycled in the process. Recycling of a gaseous reactor coolant medium often involves recompression to feed one or more of the reactors. Compressors and associated equipment add greatly to capital and operational costs. In the present method the coolant medium is preferably selected to completely dissolve ethylene. In this case the coolant medium only requires a single reactor and a condenser, whereas a simple recycle pump is sufficient. Thus expensive recycling, such as the use of an expensive recycle blower, is no longer required, which adds further to the advantages of the present method.

The invention is illustrated by the following Figures, which are not meant to limit the invention in any way, showing a scheme of an apparatus that can be used for performing the process of the invention.
Fig. 1 is a scheme of an apparatus for performing the method according to the invention with the heat exchanger positioned outside the reactor.
Fig. 2 is a scheme of an apparatus for performing the method according to the invention with the heat exchanger positioned inside the reactor.

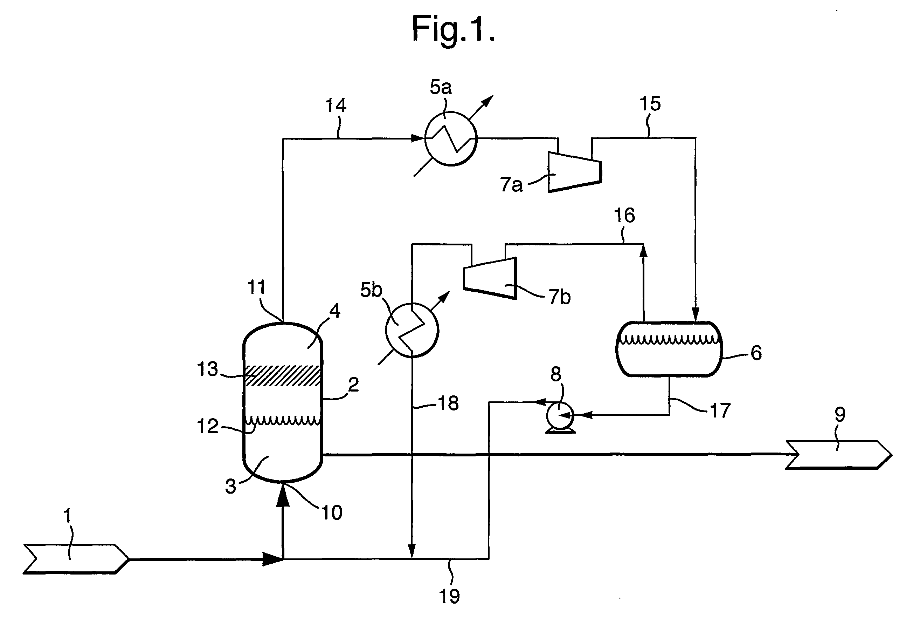

Fig. 1 shows a reactor 2 with a liquid phase 3 and a gas phase 4 being in equilibrium through gas/liquid interface 12. The liquid phase comprises ethylene, the iron complex of a 2,6-bis(arylimino)pyridine derivative, alpha-olefin oligomer, and optionally solvents and auxiliaries such as a co-catalyst. The optional solvents are selected as to dissolve ethylene. The reactor contains an inlet 10 through which the reactor feed 1 is transported, a gas outlet 11, and a reactor bottom outlet 9. In the embodiment of Figure 1, outlet 11 is connected through a conduit 14 to heat exchanger 5a, which is connected through conduit 15 to gas-liquid separator 6. If necessary, conduit 15 may contain a compressor 7a. Gas-liquid separator 6 has an outlet 17 for transporting the liquid, optionally through a pump 8, to obtain a pressurized liquid stream 17 that is recycled via conduit 19 to reactor 2. The gas leaves the gas-liquid separator 6 through conduit 16, which may optionally comprise compressor 7b and/or heat exchanger 5b, to obtain a cooled gas stream 18 that is recycled to reactor 2. If no condensation occurs in conduit 15, gas-liquid separator 6, and pump 8 are redundant and may be deleted. In that case conduit 15 can directly be connected to compressor 7b and/or heat exchanger 5b, if present, or to conduit 19. Reactor 2 may contain an optional entrainment separator 13.

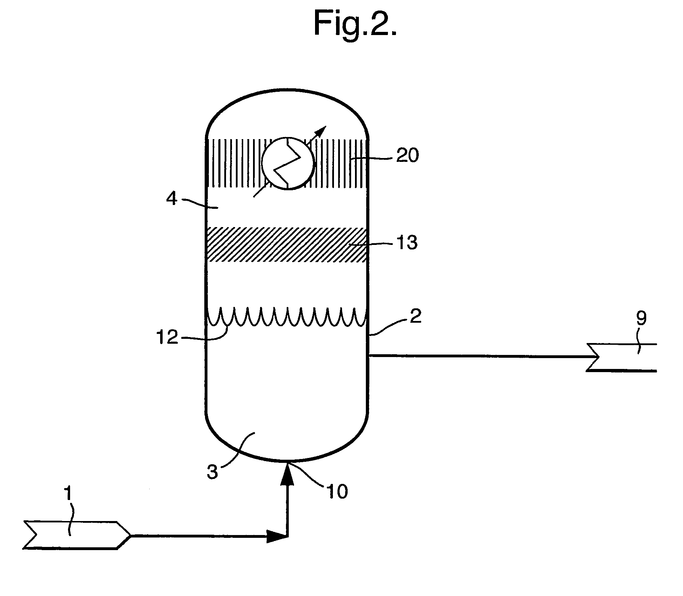

Fig. 2 shows another embodiment according to the invention. In this embodiment the reactor feed 1 is introduced into the reactor 2 through inlet 10. The liquid phase 3 in the reactor is in equilibrium with the gas phase 4 through gas/liquid interface 12. In the section of the reactor containing the gas phase 6, a heat exchanger 20 is placed, which is not in contact with the liquid phase 3. The section of the gas phase 6 may optionally contain an entrainment separator 13. The heat exchanger 20 cools the gas, after which at least part of the gas condenses and the cooled condensate falls down from the surface of the heat exchanger 20 into the liquid phase 3, thereby cooling the liquid medium. The reaction product may then be discharged from the reactor through the reactor bottom outlet 9.

Hence according to a further aspect of the present invention there is provided an apparatus for performing the process of making linear alpha-olefin oligomer described above, comprising a reactor (2), which can accommodate a liquid (3) and a gas (4) phase, an inlet (10) through which the reactor feed (1) can be transported, a reactor bottom outlet (9), and at least one heat exchanger (5a,b;20), which is positioned as to prevent direct contact with the liquid phase (3), and further optionally a gas outlet (11), pumps (8), compressors (7a,b), an entrainment separator (13), and/or a gas-liquid separator (6).

## Claims

1. A process for making a linear alpha-olefin oligomer in a reactor comprising a liquid and a gas phase, comprising the steps of catalytically oligomerizing ethylene in the presence of an iron complex of a 2,6-bis(arylimino)pyridine derivative, to the alpha-olefin oligomer with an average molecular weight between 50 and 350 under release of heat, and removing the heat with a heat exchanger, which is not in direct contact with the liquid phase, using at least part of the gas phase as a coolant medium.

2. The process according to claim 1 wherein an aluminum-based co-catalyst, preferably a methylaluminoxane, is added to the liquid phase.

3. The process according to claim 1 or 2 wherein the average molecular weight is between 60 and 280, more preferably between 80 and 210.

4. The process according to any one of claims 1 to 3 wherein one of the aryl moieties of the 2,6-bis(arylimino)pyridine derivative is 2,6-disubstituted with the group CH₂R or C₂H₅R, wherein R is selected from H and F, and the other aryl moiety is 2,6-unsubstituted, or wherein both aryl moieties of the 2,6-bis(arylimino)pyridine derivative are 2,6-disubstituted with F or Cl.

5. The process according to any one of claims 1 to 4 wherein the 2,6-bis(arylimino)pyridine derivative has the formula: 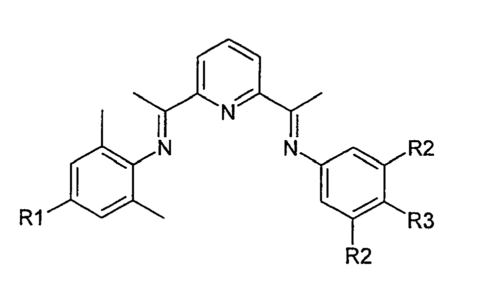 wherein
R1 is H or CH₃;
R2 is H, tert-butyl or phenyl and
R3 is H, tert-butyl or OR' wherein R' stands for CH₃, Si(CH₃)₃ or eicosyl (C₂₀H₄₁); or

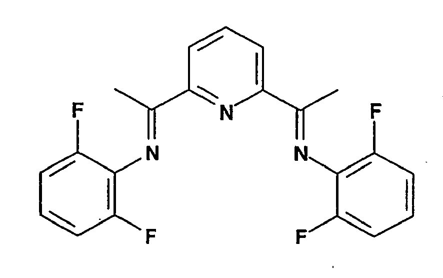

6. The process according to any one of claims 1 to 5 wherein the coolant medium is selected from an alkane, alkene, and aromatic compound, and mixtures thereof.

7. The process according to any one of claims 1 to 6 wherein the coolant medium is selected from propane, n-pentane, isopentane, ethylene, 1-butene, o-, m-, and p-xylene, and toluene, and mixtures thereof.

8. An apparatus for performing the process of making linear alpha-olefin oligomer according to any one of claims 1-7, comprising a reactor (2), which can accommodate a liquid (3) and a gas (4) phase, an inlet (10) through which the reactor feed (1) can be transported, a reactor bottom outlet (9), and at least one heat exchanger (5a,b:20), which is placed inside the reactor and which is positioned as to prevent direct contact with the liquid phase (3), an entrainment separator (13), and further optionally a gas outlet (11), pumps (8), compressors (7a,b), and/or a gas-liquid separator (6).

## Patentansprüche

1. Verfahren zur Herstellung eines linearen alpha-Olefin-Oligomers in einem Reaktor, welcher eine Flüssigkeit und eine Gasphase umfasst, umfassend die Schritte des katalytischen O-ligomerisierens von Ethylen in Gegenwart eines Eisenkomplexes eines 2,6-Bis(arylimino)pyridin-Derivats zum alpha-Olefin-Oligomer mit einem mittleren Molekulargewicht von 50 bis 350 unter Freisetzung von Wärme, und Abführen der Wärme mit einem Wärmetauscher, welcher nicht im direkten Kontakt mit der flüssigen Phase steht, wobei wenigstens ein Teil der Gasphase als ein Kühlmedium verwendet wird.

2. Verfahren nach Anspruch 1, wobei ein auf Aluminium basierender Co-Katalysator, vorzugsweise ein Methylaluminoxan, der flüssigen Phase zugesetzt wird.

3. Verfahren nach Anspruch 1 oder 2, wobei das mittlere Molekulargewicht von 60 bis 280, stärker bevorzugt von 80 bis 210 beträgt.

4. Verfahren nach einem der Ansprüche 1 bis 3, wobei einer der Arylreste des 2,6-Bis(arylimino)pyridin-Derivats mit der Gruppe CH₂R oder C₂H₅R 2,6-disubstituiert ist, wobei R unter H und F ausgewählt ist, und der andere Arylrest 2,6-unsubstituiert ist, oder wobei beide Arylreste des 2,6-Bis(arylimino)pyridin-Derivats mit F oder Cl 2,6-disubstituiert sind.

5. Verfahren nach einem der Ansprüche 1 bis 4, wobei das 2,6-Bis(arylimino)pyridin-Derivat die Formel: 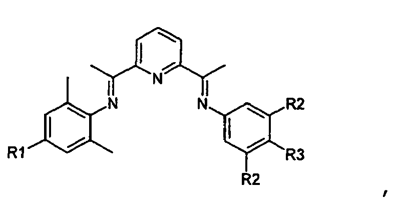 worin
R1 für H oder CH₃ steht;
R2 H, tert-Butyl oder Phenyl bedeutet, und
R3 H, tert-Butyl oder OR' darstellt, wobei R' für CH₃, Si(CH₃)₃ oder Eicosyl (C₂₀H₄₁) steht; oder 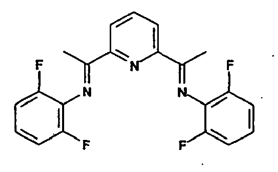
besitzt.

6. Verfahren nach einem der Ansprüche 1 bis 5, wobei das Kühlungsmedium unter einem Alkan, Alken und einer aromatischen Verbindung, und Gemischen hievon ausgewählt ist.

7. Verfahren nach einem der Ansprüche 1 bis 6, wobei das Kühlungsmedium unter Propan, n-Pentan, Isopentan, Ethylen, 1-Buten, O-, m- und p-Xylol und Toluol, und Gemischen hievon ausgewählt ist.

8. Vorrichtung zur Durchführung des Verfahrens zur Herstellung eines linearen alpha-Olefin-Oligomers gemäß einem der Ansprüche 1 bis 7, umfassend einen Reaktor (2), welcher eine flüssige Phase (3) und eine Gasphase (4) aufnehmen kann, einen Einlass (10), durch welchen das Reaktoreinsatzmaterial (1) durchgeleitet werden kann, einen Auslass (9) am Reaktorboden und wenigstens einen Wärmetauscher (5a, b; 20), welcher innerhalb des Reaktors angeordnet ist und welcher so angeordnet ist, dass der direkte Kontakt mit der flüssigen Phase (3) vermieden wird; einen Abscheider für mitgerissene Flüssigkeit (13) und ferner wahlweise einen Gasauslass (11), Pumpen (8), Kompressoren (7a, b) und/oder einen Gas-Flüssigkeits-Abscheider (6).

## Revendications

1. Procédé de fabrication d'un oligomère d'alphaoléfine linéaire dans un réacteur comprenant une phase liquide et une phase gazeuse, comprenant les étapes consistant à effectuer l'oligomérisation de l'éthylène par voie catalytique en présence d'un complexe de fer à base d'un dérivé de 2,6-bis-(arylimino)pyridine de manière à former un oligomère d'alpha-oléfine présentant un poids moléculaire moyen compris entre 50 et 350 avec libération de chaleur, et à dissiper la chaleur à l'aide d'un échangeur de chaleur qui n'est pas en contact direct avec la phase liquide, en utilisant au moins une partie de la phase gazeuse comme agent réfrigérant.

2. Procédé selon la revendication 1, dans lequel un co-catalyseur à base d'aluminium, de préférence, un méthylaluminoxane, est ajouté à la phase liquide.

3. Procédé selon la revendication 1 ou 2, dans lequel le poids moléculaire moyen est compris entre 60 et 280, mieux encore, entre 80 et 210.

4. Procédé selon l'une quelconque des revendications 1 à 3, dans lequel l'un des radicaux aryle du dérivé de 2,6-bis(arylimino)-pyridine est disubstitué au niveau des positions 2 et 6 par le groupement CH₂R ou C₂H₅R, dans lequel R est choisi parmi l'hydrogène et le fluor, et l'autre radical aryle n'est pas substitué au niveau des positions 2 et 6, ou dans lequel les deux radicaux aryle du dérivé de 2,6-bis(arylimino)pyridine sont disubstitués au niveau des positions 2 et 6 par les éléments fluor ou chlore.

5. Procédé selon l'une quelconque des revendications 1 à 4, dans lequel le dérivé de 2,6-bis(arylimino)pyridine présente la formule suivante : 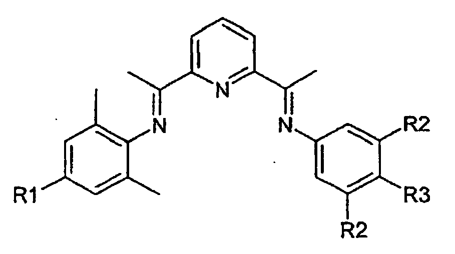 dans laquelle :
R1 représente l'hydrogène ou un groupement CH₃ ;
R2 représente l'hydrogène, un groupement tert-butyle ou phényle, et
R3 représente l'hydrogène, un groupement tert-butyle ou un groupement OR', R' représentant un groupement CH₃, Si(CH₃)₃ ou eicosyle (C₂₀H₄₁) ; ou

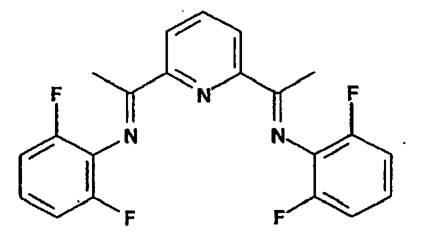

6. Procédé selon l'une quelconque des revendications 1 à 5, dans lequel l'agent réfrigérant est choisi dans le groupe constitué d'un alcane, d'un alcène et d'un composé aromatique et de leurs mélanges.

7. Procédé selon l'une quelconque des revendications 1 à 6, dans lequel l'agent réfrigérant est choisi parmi le propane, le n-pentane, l'isopentane, l'éthylène, le 1-butène, le o-, m- et p-xylène et le toluène et des mélanges de ceux-ci.

8. Appareil pour mettre en oeuvre le procédé de fabrication d'un oligomère d'alpha-oléfine linéaire selon l'une quelconque des revendications 1 à 7, comprenant un réacteur (2), qui peut recevoir une phase liquide (3) et une phase gazeuse (4), une entrée (10) à travers laquelle l'alimentation de réacteur (1) peut être délivrée, une sortie de fond de réacteur (9) et au moins un échangeur de chaleur (5a,b ; 20), qui est placé à l'intérieur du réacteur et qui est positionné de manière à empêcher un contact direct avec la phase liquide (3), un séparateur avec entraînement (13) et, éventuellement, une sortie de gaz (11), des pompes (8), des compresseurs (7a,b) et/ou un séparateur gaz-liquide (6).
